(19) Europäisches Patentamt
European Patent Office
Office européen des brevets

(11) EP 0 820 758 B1

(12) **EUROPÄISCHE PATENTSCHRIFT**

(45) Veröffentlichungstag und Bekanntmachung des Hinweises auf die Patenterteilung:
**18.01.2006 Patentblatt 2006/03**

(51) Int Cl.:
***A61K 8/06*** *(2006.01)* ***A61K 8/39*** *(2006.01)*
***A61K 8/86*** *(2006.01)* ***A61K 8/41*** *(2006.01)*
***A61Q 5/02*** *(2006.01)* ***A61Q 5/12*** *(2006.01)*

(21) Anmeldenummer: **97111504.3**

(22) Anmeldetag: **08.07.1997**

(54) **Haarpflegezubereitung in Form einer transparenten oder transluzenten Mikroemulsion vom Typ Öl-in-Wasser**

Transparent and translucent oil-in-water microemulsion for hair care

Composition pour le soin des cheveux sous la forme d'une microémulsion huile-dans-l'eau transparente ou translucide

(84) Benannte Vertragsstaaten:
**AT BE CH DE ES FR GB IT LI NL SE**

(30) Priorität: **25.07.1996 DE 19629951**

(43) Veröffentlichungstag der Anmeldung:
**28.01.1998 Patentblatt 1998/05**

(73) Patentinhaber: **Beiersdorf Aktiengesellschaft 20245 Hamburg (DE)**

(72) Erfinder:
- **Schreiber, Jörg, Dr.
22087 Hamburg (DE)**
- **Demitz, Michael
22529 Hamburg (DE)**
- **Reidel, Jan-Henric, Dr.
22307 Hamburg (DE)**

(56) Entgegenhaltungen:
**EP-A- 0 270 249** **EP-A- 0 490 053**
**DE-A- 2 710 468**

- **J. FALBE / M. REGITZ: "Römpp Chemie Lexikon" 1990 , GEORG THIEME VERLAG , STUTTGART - NEW YORK XP002144297 * Seite 1156 - Seite 1158 ***
- **J. FALBE / M. REGITZ: "Römpp Chemie Lexikon" 1992 , GEORG THIEME VERLAG , STUTTGART - NEW YORK XP002144298 * Seite 4495 - Seite 4499 ***

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 820 758 B1

Printed by Jouve, 75001 PARIS (FR)

## Beschreibung

Die vorliegende Erfindung betrifft kosmetische Haarpflegezubereitungen.

Die vorliegende Erfindung betrifft haarkosmetische Wirkstoffkombinationen und Zubereitungen, solche Kombinationen enthaltend. Insbesondere betrifft die vorliegende Erfindung haarkosmetische Wirkstoffkombinationen und Zubereitungen zur Pflege des Haars und der Kopfhaut. In einer bevorzugten Ausführungsform betrifft die vorliegende Erfindung Wirkstoffkombinationen und Zubereitungen, die dazu dienen, das einzelne Haar zu kräftigen und/oder der Haartracht insgesamt Halt und Fülle zu verleihen.

Das menschliche Haar kann, grob verallgemeinert, unterteilt werden in den lebenden Teil, die Haarwurzel, und den toten Teil, den Haarschaft. Der Haarschaft seinerseits besteht aus der Medulla, welche allerdings entwicklungsgeschichtlich bedingt für den neuzeitlichen Menschen unbedeutend geworden und zurückgebildet ist und bei dünnem Haar oft gänzlich fehlt, ferner dem die Medulla umschließenden Cortex und der die Gesamtheit aus Medulla und Cortex umhüllenden Cuticula.

Insbesondere die Cuticula, aber auch der keratinöse Bereich zwischen Cuticula und Cortex als Außenhülle des Haares sind besonderer Beanspruchung durch Umwelteinflüsse, durch Kämmen und Bürsten, aber auch durch Haarbehandlung, insbesondere Haarfärbung und Haarverformung, z.B. Dauerwellverfahren, ausgesetzt.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vortagen, nachweisbar. Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessem, beispielsweise dadurch, daß sie dem Haar mehr Fülle verleihen, die Haartracht über einen längeren Zeitraum fixieren oder seine Frisierbarkeit verbessern.

Die Eigenschaft der Fülle wird einer Frisur beispielsweise zugeschrieben, wenn das Haar nach der Behandlung nicht flach auf der Kopfhaut aufliegt und gut frisierbar ist.

Die Eigenschaft des Volumen wird einer Frisur beispielsweise zugeschrieben, wenn das Haar nach der Behandlung Fülle und Sprungkraft aufweist.

Die Eigenschaft des Body's wird einer Frisur beispielsweise zugeschrieben, wenn das Haarvolumen selbst unter äußeren, störenden Einflüssen groß bleibt.

Durch quaternäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Der Stande der Technik ließ es aber an Wirkstoffen und Zubereitungen mangeln, welche dem geschädigten Haar in befriedigender Weise Pflege zukommen ließen. Auch erwiesen sich Zubereitungen, die der Haartracht Fülle geben sollten, oft als unzureichend, zumindest waren sie ungeeignet, als Haarpflegezubereitungen eingesetzt zu werden. Die Haartracht fixierende Zubereitungen des Standes der Technik enthalten beispielsweise in der Regel viskose Bestandteile, welche Gefahr laufen, ein Gefühl der Klebrigkeit zu erwecken, welches oft durch geschickte Formulierung kompensiert werden muß.

Aufgabe war daher, den Nachteilen des Standes der Technik Abhilfe zu schaffen.

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

In einfachen Emulsionen liegen in der einen Phase feindisperse, von einer Emulgatorhülle umschlossene Tröpfchen der zweiten Phase (Wassertröpfchen in W/O- oder Lipidtröpfchen in O/W-Emulsionen) vor. Die Tröpfchendurchmesser der gewöhnlichen Emulsionen liegen im Bereich von ca 1 μm bis ca. 50 μm. Solche "Makroemulsionen" sind, ohne weitere färbende Zusätze, milchigweißgefärbt und opak. Feinere "Makroemulsionen", deren Tröpfchendurchmesser im Bereich von ca. $10^{-1}$ μm bis ca. 1 μm liegen, sind, wiederum ohne färbende Zusätze, bläulichweißgefärbt und undurchsichtig.

Mizellaren und molekularen Lösungen mit Partikeldurchmessern kleiner als ca. $10^{-2}$ μm, ist vorbehalten, klar und transparent zu erscheinen.

Der Tröpfchendurchmesser von transparenten bzw. transluzenten Mikroemulsionen dagegen liegt im Bereich von etwa $10^{-2}$ μm bis etwa $10^{-1}$ μm. Solche Mikroemulsionen sind meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe feiner dispers vorliegen können als in der dispersen Phase von "Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte im allgemeinen durch hohe kosmetische Eleganz aus.

Nachteilig an den Mikroemulsionen des Standes der Technik ist, daß stets ein hoher Gehalt an einem oder mehreren Emulgatoren eingesetzt werden muß, da die geringe Tröpfchengröße eine hohe Grenzfläche zwischen den Phasen bedingt, welche in der Regel durch Emulgatoren stabilisiert werden muß.

An sich ist die Verwendung der üblichen kosmetischen Emulgatoren unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden.

Eine besondere Aufgabe der vorliegenden Erfindung war es, feindisperse Zubereitungen vom Typ Öl-in-Wasser mit einem möglichst niedrigen Emulgatorgehalt zur Verfügung zu stellen, welche nicht die Nachteile des Standes der Technik aufweisen und welche für verschiedenste kosmetische und/oder dermatologische Anwendungen, beispielsweise die vorab beschreibenen Verwendungen finden können. Eine weitere Aufgabe der Erfindung war, das begrenzte Angebot an feindispersen Zubereitungen vom Typ Öl-in-Wasser des Standes der Technik zu bereichem.

Es ist an sich bekannt, daß hydrophile Emulgatoren, namentlich polyethoxylierte und polypropoxylierte Emulgatoren, bei steigender Temperatur ihr Löslichkeitsverhalten von wasserlöslich zu fettlöslich ändern. Ein Kennzeichen für die Hydrophilie eines gegebenen Emulgators ist dessen HLB-Wert.

Die Definition des HLB-Wertes ist für Polyolfettsäureester gegeben durch die Beziehung

$$HLB = 20 * (1 - S/A) \qquad \text{(Formel I)}$$

Für eine Gruppe von Emulgatoren, deren hydrophiler Anteil nur aus Ethylenoxideinheiten besteht, gilt die Beziehung

$$HLB = E/5 \qquad \text{(Formel II)}$$

wobei

S = Verseifungszahl des Esters,
A = Säurezahl der zurückgewonnen Säure
E = Massenanteil Ethylenoxid (in %) am Gesamtmolekül

bedeuten.

Emulgatoren mit HLB-Werten von 6-8 sind im allgemeinen W/O-Emulgatoren, solche mit HLB-Werten von 8-18 sind im allgemeinen O/W-Emulgatoren.

Literatur: "Kosmetik - Entwicklung, Herstellung und Anwendung kosmetischer Mittel"; W.Umbach (Hrsg.), Georg Thieme Verlag 1988.

Der Temperaturbereich, in dem die Emulgatoren ihre Löslichkeit änderen, wird Phaseninversionstemperaturbereich genannt. Für den Phaseninversionstemperaturbereich soll innerhalb dieser Schrift auch die Abkürzung "PIT"' gebraucht werden.

Die Änderung dieses Löslichkeitsverhaltens äußert sich bekanntermaßen darin, daß eine Mischung aus Wasser, Öl und O/W-Emulgatoren, welche unterhalb des PIT nach Rühren eine O/W-Emulsion ergibt, auf eine Temperatur oberhalb des PIT gebracht wird, typischerweise etwa 70-90° C, als Zwischenstufe den Zustand einer Mikroemulsion durchlaufen kann, um schließlich oberhalb des PIT eine W/O-Emulsion zu ergeben. Wird diese Emulsion abgekühlt, wird wieder eine O/W Emulsion erhalten, welche aber eine Tröpfchengröße von bis zu 200 nm besitzt.

Auf solche Weise hergestellte Mikroemulsionen des Standes der Technik haben allerdings den Nachteil, daß

die Tröpfchengröße immer noch recht hoch ist, daß die Emulsion bei Raumtemperatur opak weiß bis bläulich ist.

Weiterhin ist nachteilig, daß auf solche Weise hergestellte Mikroemulsionen zwar bei hoher Temperatur, also beispielsweise im PIT, praktisch transparent sein können, aber beim Absinken auf Raumtemperatur wieder undurchsichtig werden.

Auch diesen Übelständen galt es also, abzuhelfen.

Erstaunlicherweise werden all diese Aufgaben gelöst durch Haarpflegezubereitungen, in Form einer transparenten oder transluzenten Mikroemulsion vom Typ Öl-in-Wasser, **die einen Emulgatorgehalt kleiner als 2,0 Gew.-%, bezogen auf das Gesamtgewicht der Emulsion, aufweisen,**

- umfassend eine Ölphase, und eine Wasserphase
- enthaltend:

einen oder mehreren polyethoxylierte O/W-Emulgatoren und/oder
einen oder mehrere polypropoxylierte O/W-Emulgatoren und/oder
einen oder mehrere polyethoxylierte und polypropoxylierte O/W-Emulgatoren, gewählt aus der Gruppe

- der Fettalkoholethoxylate der allgemeinen Formel R-O-(-$CH_2$-$CH_2$-O-)-H, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel R-O-(-$CH_2$-$CH_2$-O-)-R',

wobei R und R' unabhängig voneinander verzweigte oder unverzweigte Alkyl- oder Alkenylreste und n eine Zahl von 10 bis 80 darstellen

- der Fettsäureethoxylate der allgemeinen Formel R-COO-(-$CH_2$-$CH_2$-O-)-H, wobei R einen verzweigten oder unverzweigten Alkyl- oder Alkenylrest und n eine Zahl von 10 bis 40 darstellen,
- der veretherten Fettsäureethoxylate der allgemeinen Formel R-COO-(-$CH_2$-$CH_2$-O-)-R', wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel R-COO-(-$CH_2$-$CH_2$-O-)-C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweiger Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel R-O-(-$CH_2$-$CH_2$-O-)-$CH_2$-COOH bzw. deren kosmetisch oder pharmazeutisch akzeptablen Salze, wobei R einen verzweigten oder unverzweigten Alkyloder Alkenylrest mit 5-30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel R-O-(-$CH_2$-$CH_2$-O-)-$SO_3$-H mit kosmetisch oder pharmazeutisch akzeptablen Kationen, wobei R einen verzweigten oder unverzweigten Alkyl- oder Alkenylrest mit 5-30 C-Atomen und n eine Zahl von 1 bis 50 darstellen.
- der Fettalkohol propoxylate der allgemeinen Formel R-O-(-$CH_2$-$CH(CH_3)$-O-)$_n$-H, wobei R einen verzweigten oder unverzweigten Alkyl- oder Alkenylrest und n eine Zahl von 10 bis 80 darstellen,
- der Polypropylenglycolether der allgemeinen Formel R-O-(-$CH_2$-$CH(CH_3)$-O-)$_n$-R', wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel R-COO-(-$CH_2$-$CH(CH_3)$-O-)$_n$-R', wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel R-COO-(-$CH_2$-$CH(CH_3)$-O-)$_n$-C(O)-R', wobei R und R' unabhängig voneinander verzweigte oder unverzweigte Alkyl- oder Alkenylreste und n eine Zahl von 10 bis 80 darstellen,

- der Fettsäurepropoxylate der allgemeinen Formel $R-COO-(-CH_2-CH(CH_3)-O-)_n-H$, wobei R einen verzweigten oder unverzweigten Alkyloder Alkenylrest und n eine Zahl von 10 bis 80 darstellen,
- der Polypropylenglycolglycerinfettsäureester gesättigter und/oder ungesättigter, verzweigter und/oder unverzweiger Fettsäuren und einem Propoxylierungsgrad zwischen 3 und 80
- der propoxylierten Sorbitanester mit einem Propoxylierungsgrad von 3 bis 100
- der Cholesterinpropoxylate mit einem Propoxylierungsgrad von 3 bis 100
- der propoxylierten Triglyceride mit einem Propoxylierungsgrad von 3 bis 100 der Alkylethercarbonsäuren der allgemeinen Formel $R-O-(-CH_2-CH(CH_3)O-)_n-CH_2-COOH$ bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel $R-O-(-CH_2-CH(CH_3)-O-)_n-SO_3-H$ mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylate/propoxylate der allgemeinen Formel $R-O-X_n-Y_m-H$,

wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,

- der Polypropylenglycolether der allgemeinen Formel $R-O-X_n-Y_m-R'$, wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel $R-COO-X_n-Y_m-R'$, wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel $R-COO-X_n-Y_m-H$, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

**wobei der Anteil der polyethoxylierten bzw. der polypropoxylierten bzw. der polyethoxylierten und polypropoxylierten Emulgatoren unter 1,5 Gew.-%, bezogen auf das Gesamtgewicht der Zubereitung liegt**

- gewünschtenfalls ferner enthaltend einen oder mehrere W/O-Emulgatoren
- erhältlich durch ein Verfahren, dergestalt, daß

- ein Gemisch aus den Grundkomponenten,
  - welches unterhalb des Phaseninversionstemperaturbereiches eine O/W-Emulsion bildet,
  - auf eine Temperatur oberhalb oder innerhalb des Phaseninversionstemperaturbereiches gebracht,
  - und die gebildete Mikroemulsion hernach auf Raumtemperatur abkühlt wird,

- ferner enthaltend mindestens ein kationische Tensides, gewählt aus der Gruppe der quaternären Ammoniumverbindungen, sofern diese keine flimbildenden Eigenschaften besitzen, insbesondere Benzyldialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylam inoxide, **wobei weniger als 0,5 Gew.-% an einem oder mehreren kationischen Tensiden vorliegen.**

Erfindungsgemäße Zubereitungen haben eine niedrige Viskosität, sind versprühbar, eignen sich vorzüglich als Vehikel für verschiedenste Wirkstoffe, insbesondere lipidlösliche Wirkstoffe und zeichnen sich darüberhinaus durch vorzügliche Haut-, Haar-und Schleimhautverträglichkeit aus.

Zwar wird in der JP-A-Hei-06/262060 (gem. Patent Abstracts of Japan) eine solubilisierte Zubereitung beschrieben, welche beispielsweise Polyethylenglycolalkylether umfassen kann. Die Ölphase der offenbarten Beispiele betrifft allerdings das leichtflüchtige Heptan, welches landläufig kaum als Ölkomponente, schon gar keine kosmetische oder pharmazeutische Ölkomponente angesehen werden kann. Die gemäß dieser Lehre gefertigten Mikroemulsionen können trotz des vorgetragenen Anspruches nicht als Kosmetika oder Pharmaka angesehen werden.

In der US-A-4,146,499 werden ferner Mikroemulsiorien beschrieben, welche zwar ethoxylierte Rohstoffe enthalten, bei welcher allerdings die Ölphase typischerweise von solch unphysiologischen Bestandteile wie Benzol, Tetrachlormethan, Dichlormethan und Fluorchlorkohlenwasserstoffe dargestellt wird. Auch dieses Dokument des Standes der Technik konnte mithin nicht den Weg zur vorliegenden Erfindung weisen.

In der EP-B-490 053 werden Haarkurmittel in Form einer Mikroemulsion beschrieben, welche dadurch gekennzeichnet sind, daß sie zwischen 5,5 und 30 Gew.-% an Tensiden enthalten. Auch dieses Dokument des Standes der Technik konnte nicht den Weg zur vorliegenden Erfindung weisen

Insbesondere ist vorteilhaft, wenn der polyethoxylierte bzw. polypropoxylierte bzw. polyethoxylierte und polypropoxylierte O/W-Emulgator oder die polyethoxylierten bzw. polypropoxylierten bzw. polyethoxylierten und polypropoxylierten O/W-Emulgatoren gewählt wird oder werden aus der Gruppe

- der Fettalkoholethoxylate der allgemeinen Formel $R-O-(-CH_2-CH_2-O-)_n-H$, wobei R einen verzweigten oder unverzweigten Alkyl- oder Alkenylrest mit 5-30 C-Atomen und n eine Zahl von 10 bis 25 darstellen,
- der ethoxylierten Wollwachsalkohole mit HLB-Werten von 11-16. ganz besonders vorteilhaft mit mit HLB-Werten von 14 -16,
- der Polyethylenglycolether der allgemeinen Formel $R-O-(-CH_2-CH_2-O-)_n-R'$, wobei R und R' unabhängig voneinander verzweigte oder unverzweigte Alkyl- oder Alkenylreste mit 5 - 30 C-Atomen und n eine Zahl von 10 bis 40 darstellen,
- der Fettsäureethoxylate der allgemeinen Formel $R-COO-(-CH_2-CH_2-O-)_n-H$, wobei R einen verzweigten oder unverzweigten Alkyl- oder Alkenylrest mit 5 - 30 C-Atomen und n eine Zahl von 10 bis 30 darstellen,
- der veretherten Fettsäureethoxylate der allgemeinen Formel $R-COO-(-CH_2-CH_2-O-)_n-R'$, wobei R und R' unabhängig voneinander verzweigte oder unverzweigte Alkyl- oder Alkenylreste mit 5 - 30 C-Atomen und n eine Zahl von 10 bis 50 darstellen,
- der veresterten Fettsäureethoxylate der allgemeinen Formel $R-COO-(-CH_2CH_2-O-)_n$ -C(O)-R', wobei R und R' unabhängig voneinander verzweigte oder unverzweigte Alkyl- oder Alkenylreste mit 5 - 30 C-Atomen und n eine Zahl von 10 bis 50 darstellen,
- der Polyethylenglycolglycerinfettsäureester gesättigter und/oder ungesättigter, verzweigter und/oder unverzweiger Fettsäuren mit 6 bis 26 C-Atomen und einem Ethoxylierungsgrad zwischen 3 und 40
- der ethoxylierten Sorbitanester mit einem Ethoxylierungsgrad von 3 bis 30
- der Cholesterinethoxylate mit HLB-Werten von 11 - 16, ganz besonders vorteilhaft mit HLB-Werten von 14 - 16
- der ethoxylierten Triglyceride mit HLB-Werten von 11 - 16, ganz besonders vorteilhaft mit mit HLB-Werten von 14 - 16
- der Alkylethercarbonsäuren der allgemeinen Formel $R-O-(-CH_2-CH_2-O-)_nCH_2-COOH$ bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 20 darstellen,
- der Polyoxyethylensorbitolfettsäureester, basierend auf verzweigten oder unverzweigten Alkan- oder Alkensäuren und einen Ethoxylierungsgrad von 10 bis 80 aufweisend, beispielsweise vom Sorbeth-Typ,
- der Alkylethersulfate bzw. die diesen Sulfaten zugrundeliegenden Säuren der allgemeinen Formel $R-O-(-CH_2-CH_2-O-)_n-SO_3-H$ mit kosmetisch oder pharmazeutisch akzeptablen Kationen, wobei R einen verzweigten oder unverzweigten Alkyl- oder Alkenylrest mit 5-30 C-Atomen und n eine Zahl von 3 bis 30 darstellen,
- der Fettalkoholpropoxylate der allgemeinen Formel $R-O-(-CH_2-CH(CH_3)-O-)_n-H$. wobei R einen verzweigten oder unverzweigten Alkyloder Alkenylrest mit 5 - 30 C-Atomen und n eine Zahl von 10 bis 30 darstellen,
- der Polypropylenglycolether der allgemeinen Formel $R-O-(-CH_2-CH(CH_3)-O-)_n-R'$, wobei R und R' unabhängig voneinander verzweigte oder unverzweigte Alkyl- oder Alkenylreste mit 5 - 30 C-Atomen und n eine Zahl von 10 bis 40 darstellen,
- der propoxylierten Wollwachsalkohole mit HLB-Werten von 11 - 16, ganz besonders vorteilhaft mit HLB-Werten von 14,5 - 15,5,
- der Fettsäurepropoxylate der allgemeinen Formel $R-COO-(-CH_2-CH(CH_3)-O-)_n-H$, wobei R einen verzweigten oder unverzweigten Alkyloder Alkenylrest mit 5 - 30 C-Atomen und n eine Zahl von 10 bis 40 darstellen,
- der veretherten Fettsäurepropoxylate der allgemeinen Formel $R-COO-(-CH_2-CH(CH_3)-O-)_n-R'$, wobei R und R' unabhängig voneinander verzweigte oder unverzweigte Alkyl- oder Alkenylreste mit 5 - 30 C-Atomen und n eine Zahl von 10 bis 30 darstellen,

- der veresterten Fettsäurepropoxylate der allgemeinen Formel $R-COO-(-CH_2-CH(CH_3)-O-)_n-C(O)-R'$, wobei R und R' unabhängig voneinander verzweigte oder unverzweigte Alkyl- oder Alkenylreste mit 5 - 30 C-Atomen und n eine Zahl von 10 bis 50 darstellen,
- der Polypropylenglycolglycerinfettsäureester gesättigter und/oder ungesättigter, verzweigter und/oder unverzweiger Fettsäuren mit 6 bis 26 C-Atomen und einem Propoxylierungsgrad zwischen 3 und 50
- der propoxylierten Sorbitanester mit einem Propoxylierungsgrad von 3 bis 80
- der Cholesterinpropoxylate mit HLB-Werten von 11 - 16, ganz besonders vorteilhaft mit mit HLB-Werten von 14,5 - 15,5
- der propoxylierten Triglyceride mit HLB-Werten von 11 - 16, ganz besonders vorteilhaft mit mit HLB-Werten von 14,5 - 15,5
- der Alkylethercarbonsäuren der allgemeinen Formel $R-O-(-CH_2-CH(CH_3)O-)_n-CH_2-COOH$ bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 30 darstellen,
- der Alkylethersulfate bzw. die diesen Sulfaten zugrundeliegenden Säuren der allgemeinen Formel $R-O-(-CH_2-CH(CH_3)-O-)_n-SO_3-H$ mit kosmetisch oder pharmazeutisch akzeptablen Kationen, wobei R einen verzweigten oder unverzweigten Alkyloder Alkenylrest mit 5 - 30 C-Atomen und n eine Zahl von 1 bis 30 darstellen.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 16, ganz besonders vorteilhaft mit mit HLB-Werten von 14 - 16, sofern die O/W Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylengtycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),

Polyethytenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16). Polyethylenglycol(17)cetylether (Ceteth-17), Polyethylenglycol(18)cetylether (Ceteth-18), Polyethylenglycol(19)cetylether (Ceteth-19). Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat. Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Als fakultative, dennoch erfindungsgemäß vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat. Glycerylmonocaprinat, Glycerylmonocaprylat.

Die Ölphase der erfindungsgemäßen Mikroemulsionen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Es kann auch gegebenenfalls vorteilhaft sein, Wachse, beispielsweise Cetylpalmitat, als alleinige Lipidkomponente der Ölphase einzusetzen. In solchen Fällen können die erfindungsgemäßen O/W-Mikroemulsionen auch gegebenenfalls als Mikrodispersionen fester Wachs partikel anfallen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Es wird allerdings bevorzugt, die Ölphase der erfindungsgemäßen Zubereitungen aus der Gruppe der cyclischen und/oder linearen Silikone zu wählen. Diese können als Monomere vorliegen, welche in der Regel durch Strukturelemente charakterisiert sind wie folgt:

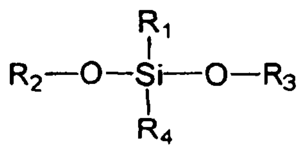

Als erfindungsgemäß vorteilhaft einzusetzenden linearen Silicone mit mehreren Siloxyleinheiten werden im allgemeinen durch Strukturelemente charakterisiert wie folgt:

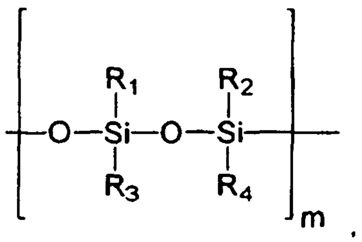

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). m kann dabei Werte von 2 - 200.000 annehmen.

Solche erfindungsgemäß vorteilhaft einzusetzenden cyclischen Silicone werden im allgemeinen durch Strukturelemente charakterisiert wie folgt

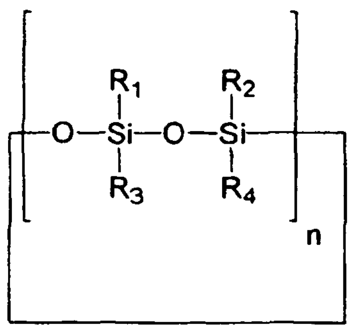

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan. Polydimethylsiloxan. Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind.

Dazu zählen beispielsweise Polysiloxan-polyalkyl-polyether-copolymere wie das Cetyl-Dimethicon-Copolyol,

Das (Cetyl-Dimethicon-Copolyol (und) Polyglyceryl-4-Isostearat (und) Hexyllaurat)

Die erfindungsgemäß verwendeten kationischen Tenside können bevorzugt gewählt werden aus der Gruppe der quaternären Ammoniumverbindungen, sofern diese keine filmbildenden Eigenschaften besitzen, insbesondere Benzyltrialkylammoniumchloride oder - bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Es kann sich erfindungsgemäß als besonders vorteilhaft erweisen, wenn die Silikonöle in der Form derivatisiert sind, daß sie eine oder mehrere quatemäre Ammoniumgruppen in einer oder mehreren organischen Resten aufweisen, beispielsweise diquaternäre Polydimethyldiloxane wie Quaternium-80 oder Dimethicon-propylbetaïn und dergleichen mehr.

Erfindungsgemäße Zubereitungen können neben den erfindungsgemäß obligatorischen kationischen Tensiden auch anionische, nichtionische und/oder amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaïne, Sulfosuccinate, Sulfobemsteinsäurehalbester, Alkylethercarboxylate, Eiweiß-Fettsäure-Kondensate, Alkylbetaïne und Amidobetaïne, Fettsäurealkanolamide, Polyglycolether-Derivate.

Erfindungsgemäße Zubereitungen zur Haarpflege können auch gegebenenfalls vorteilhaft zur Reinigung der Haar und der Kopfhaut verwendet werden.

Kosmetische Zubereitungen, die kosmetische Reinigungszubereitungen für die Haare bzw. die Kopfhaut darstellen, können in flüssiger oder halbfester Form vorliegen, beispielsweise als Gele. Die Zubereitungen enthalten mindestens eine kationische oberflächenaktive Substanz oder Gemische daraus. Sie können ferner gewünschtenfalls anionisch, nicht-ionische und/oder amphotere oberflächenaktive Substanzen oder Gemische daraus, gegebenenfalls Elektrolyte und Hilfsmittel, wie sie üblicherweise dafür verwendet werden.

Kosmetische Zubereitungen, die ein Shampoonierungsmittel darstellen, enthalten vorzugsweise zusätzlich mindestens eine anionische, nicht-ionische oder amphotere oberflächenaktive Substanz oder Gemische daraus, gegebenenfalls Elektrolyte und Hilfsmittel, wie sie üblicherweise dafür verwendet werden.

Die erfindungsgemäßen für die Reinigung des Haares bzw. der Kopfhaut vorgesehenen Zubereitungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die erfindungsgemäßen Zubereitungen haben, trotz ihres Ölgehaltes, in erstaunlicher Weise sehr gute Schaumentwicklung, hohe Reinigungskraft und wirken in hohem Maße regenerierend in bezug auf den allgemeinen Hautzustand, Insbesondere wirken die erfindungsgemäßen Zubereitungen hautglättend, vermindern das Trockenheitsgefühl der Haut und machen die Haut geschmeidig.

Derartige Ausführungsformen der erfindungsgemäßen Zubereitungen pflegen durch Umwelteinflüsse geschädigtes oder strapaziertes Haar bzw. beugen solchen Umwelteinflüssen vor. Ferner verleihen die erfindungsgemäßen Zubereitungen der Haartracht lockere Fülle und Festigkeit, ohne klebrig zu wirken. Sie dienen der Erhöhung der Haarfülle, zur Verbesserung des Haarbodys und des Haarvolumens sowie des Haltes der Haartracht.

Die Herstellung der erfindungsgemäßen Mikroemulsionen erfolgt vorteilhaft dergestalt, daß ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe, welche unterhalb des Phaseninversionstemperaturbereiches eine O/W-Emulsion bilden, auf eine Temperatur oberhalb oder innerhalb des Phaseninversionstemperaturbereiches bringt, und die gebildete Mikroemulsion hernach auf Raumtemperatur abkühlt. Dies geschieht bevorzugt unter Rühren.

Es ist jeweils möglich, auf einen Homogenisierungsschritt zu verzichten.

Das Verfahren zur Herstellung von O/W-Mikroemulsionen gemäß Anspruch 1 wird vorteilhafterweise dadurch gekennzeichnet, daß

(a) die Anfangskonzentrationen der Ölphase, der Wasserphase und gewünschtenfalls eines oder mehrerer W/O-Emulgatoren gewählt werden und diese Bestandteile zueinander gegeben werden,
(b) die Anfangskonzentration des oder der O/W-Emulgatoren, welche auch gegebenenfalls gleich Null sein kann, gewählt wird und dieser oder diese O/W-Emulgatoren zu dem in (a) erhaltenden Gemisch gegeben werden
(c) wobei das in (b) erhaltene Gemisch eine Ausgangstemperatur besitzt
(d) das in (b) erhaltende Gemisch durch geeignete Variation mindestens eines Parameters, gewählt aus der Gruppe Temperatur und der Konzentration bzw. Konzentrationen mindestens eines der gewählten Emulgatoren und/oder der Ölphase und/oder der Wasserphase, das so gebildete Gemisch den Phaseninversionsbereich zwischen

W/O-Emulsionen und O/W-Emulsionen durchläuft und in den Bereich gebracht wird, wo das Gemisch als O/W-Emulsion bzw. O/W-Mikroemulsion vorliegt,
(e) das in (d) erhaltene Gemisch sodann gegebenenfalls weiteren Aufbereitungsschritten unterworfen wird.

Erfindungsgemäß gleichermaßen vorteilhaft sind Verfahren, bei welchen die Variation des Parameters oder der Parameter darin besteht, daß

(d1) bei vorgegebener Konzentration des O/W-Emulgators bzw. der Vielzahl an O/W-Emulgatoren sowie der Wasserphase und der Ölphase die Temperatur des Gemisches variiert wird, bzw. daß
(d2) bei vorgegebener Temperatur die Konzentration mindestens eines O/W-Emulgators, bzw. daß
(d3) bei vorgegebener Temperatur und vorgegebener Konzentration mindestens eines O/W-Emulgators, die Konzentration der Ölphase und oder die Konzentration der Wasserphase variiert wird.

Es kann erfindungsgemäß gegebenenfalls bevorzugt sein, mehrere Parameter gleichzeitig oder nacheinander zu variieren.
Erfindungsgemäß können vorteilhafte haarpflegenden Zubereitungen erhalten werden, wenn der Anteil der polyethoxylierten bzw. der polypropoxylierten bzw. der polyethoxylierten und polypropoxylierten Emulgatoren unter 1,5 Gew.-%, insbesondere unter 1,0 Gew.-%, bezogen auf das Gesamtgewicht der Zubereitung liegt, und weniger als 0,5 Gew.% an einem oder mehreren kationischen Tensiden vorliegen.
In Fig. 1 wird eine stark vereinfachte Darstellung eines Phasendiagrammes wiedergegeben. Der variable Parameter P wird gegen die Temperatur θ als zweite Variable aufgetragen. P stellt dabei einen Konzentrationsparameter dar, entweder den Anteil der Ölphase, den Anteil der Wasserphase oder die Konzentration eines Emulgators oder eines Emulgatorgemisches. Für erfindungsgemäße Systeme gilt dabei, daß bei niedrigeren Temperaturen eine O/W-Emulsion vorliegt und bei Erhöhung der Temperatur der Phaseninversionsbereich durchlaufen werden kann. Bei weiterer Erhöhung der Temperatur werden W/O-Emulsionen beobachtet. Die Struktur des Systems im Phaseninversionsbereich ist dem Anschein nach nicht kritisch für die vorliegende Erfindung. Denkbar ist beispielsweise, daß im Phaseninversionsbereich lamellare Phasen, bikontinuierliche Phasen, kubische, hexagonale bzw. invers hexagonale Phasen vorliegen, auch daß der Phaseninversionsbereich aus mehreren gleichartigen oder mehr oder weniger unterschiedlichen Phasen zusammengesetzt ist.
Der Phaseninversionsbereich läßt sich mathematisch darstellen als Punktmenge innerhalb des geradlinigen Koordinatensystems Σ, welches durch die Größen Temperatur, der Konzentration eines geeigneten Emulgators bzw. eines Emulgatorengemisches in der Zubereitung sowie die jeweiligen Konzentrationen der Ölphase und der Wasserphase gebildet wird, gemäß:

$$\Sigma = \{O, \theta, m, H, W\},$$

mit

O - Koordinatenursprung
θ - Temperatur
m - Konzentration des Emulgators/Emulgatorengemisches
H - Konzentration der Ölphase
W - Konzentration der Wasserphase

Dabei muß genaugenommen natürlich in einem mehrkomponentigen Emulgatorensystern der Beitrag $m_i$ jedes einzelnen Emulgators zur Gesamtfunktion berücksichtigt werden, was bei einem i-komponentigen Emulgatorensystem zur Beziehung

$$\Sigma = \{ O, \theta, m_1, m_2, ..., m_i, H, W\} \quad \text{führt.}$$

Der Phaseninversionsbereich Φ stellt dabei im mathematischen Sinne ein zusammenhängendes Gebiet oder eine Vielzahl zusammenhängender Gebiete innerhalb des Koordinatensystems Σ dar. Φ repräsentiert die Gesamtmenge der Koordinatenpunkte K(θ, a, $m_1$, $m_2$, ..., $m_i$, H, W), welche erfindungsgemäße Gemische aus Wasserphase der Konzentration W, Ölphase der Konzentration H, i erfindungsgemäßen Emulgatoren der Konzentration $m_i$ bei der Temperatur

θ bestimmen, und für welche gilt, daß beim Übergang von einer Koordinate $K_1 \notin \Phi$ zu einer Koordinate $K_2 \in \Phi$ Phaseninversion eintritt, wie in Fig. 2 beschrieben.

Unerheblich ist dabei, ob der Phaseninversionsbereich eines gegebenen Systems ein einziges zusammenhängendes (i + 3)-dimensionalen Gebiet darstellt oder aus mehreren zusammenhängenden, aber voneinander getrennten solchen Gebieten besteht, also mehreren Phaseninversionsbereichen eines gegebenen Systems entsprechend. Im Rahmen der hiermit vorgelegten Offenbarung wird daher stets verallgemeinernd von "dem" oder "einem" Phaseninversionsbereich gesprochen, auch bei Vorliegen zweier oder mehrerer voneinander getrennter solcher Bereiche.

Als variable Koordinaten in Fig.2 werden Temperatur θ und die der vorgeschilderte Konzentrationsparameter P angegeben, wobei offengelassen bleiben kann, um welchen speziellen Konzentrationsparameter es sich handelt. Beim Übergang von $K_1$ nach $K_2$ wird lediglich die Temperatur erhöht, die anderen Variablen werden konstant gehalten.

Unter den erfindungsgemäßen Bedingungen ist dieser Prozeß nicht reversibel, d.h., kehrt das System von der Koordinate $K_2 \in \Phi$ wieder zur Koordinate $K_1 \notin \Phi$ zurück, können erfindungsgemäße transparente O/W-Mikroemulsionen erhalten werden.

Die Praxis der Herstellung einer erfindungsgemäßen Mikroemulsion besteht demgemäß vorteilhaft darin, nach Auswahl geeigneter Rohstoffe, d.h., Wasser- und Ölphase, ein oder mehrere erfindungsgemäß verwendete O/W-Emulgatoren, letzterer oder letztere vorliegend in Konzentrationen, bei welchen Phaseninversion für das gegebene Gemisch möglich ist, und gegebenenfalls weitere Substanzen, die Einzelkomponenten unter Rühren zusammenzugeben, durch Erhöhung der Temperatur des Gemisches Phaseninversion herbeizuführen, hernach unter fortwährendem Rühren das Gemisch auf Raumtemperatur abkühlen zu lassen.

Es ist dabei aber auch möglich, mehrere Parameter zugleich zu variieren, wie in Fig.3 angezeigt. In Fig. 3 wird die Konzentration der Wasserphase gegen die Temperatur aufgetragen. Ausgehend von der Koordinate $K_1 \notin \Phi$ können durch Erhöhung der Temperatur, unter Beibehaltung aller anderen Parameter, die Koordinaten $K_2 \notin \Phi$ und $k_4 \notin \Phi$ erreicht werden bzw. $K_3 \in \Phi$. Ausgehend von den Koordinaten $K_3$ und $K_4$ können durch Senken der Temperatur, unter Beibehaltung aller anderen Parameter, zurück zur Koordinate $K_1$ erfindungsgemäße O/W-Mikroemulsionen erhalten werden.

Ausgehend von den Koordinaten $K_3$ und $K_4$ kann durch Senken der Temperatur, und durch zusätzliche Variation der Konzentration der Ölphase, in Fig.3 durch Zugabe von Wasser, die Koordinate $K_5$ erreicht und können erfindungsgemäße 0/W-Mikroemulsionen erhalten werden.

Es ist angesichts der Fig. 3 konsequent, daß, ausgehend von der Koordinate $K_4$, obwohl dieser außerhalb des Phaseninversionsbereiches befindlich ist, Systeme erhalten werden können, ähnlich denen, die von $K_3$ ausgehen, da ja auch ausgehend von $K_4$ bei Senkung der Temperatur der Phaseninversionsbereich zwangsläufig durchschritten werden muß.

Auch ausgehend von der Koordinate $K_1$ kann durch Variation der Konzentration der Wasserphase, also beispielsweise durch Zugabe von Wasser, wie in Fig. 3 aufgeführt, die Koordinate $K_5$ erreicht und können erfindungsgemäße O/W-Mikroemulsionen erhalten werden. Dazu muß allerdings vorausgeschickt werden, daß in diesem Falle bereits eine erfindungsgemäße O/W-Mikroemulsion, gewissermaßen als Konzentrat, vorliegen muß, welches dann durch Verdünnen zu einer erfindungsgemäßen O/W-Mikroemulsion veränderter Zusammensetzung umgesetzt wird.

Es war jedoch nach allem erstaunlich, daß auch ausgehend von der Koordinate $K_2$, welche außerhalb des Phaseninversonsbereiches liegt, sei es bei einfacher Variation der Temperatur zurück zur Koordinate $K_1$ oder zusätzlicher Variation der Konzentration der Ölphase, also beispielsweise durch zusätzliches Verdünnen mit einer Wasserphase zur Koordinate $K_5$, erfindungsgemäße O/W-Mikroemulsionen erhältlich sind, ohne daß Phaseninversion durchlaufen wird. Dies geschieht vorteilhaft auf die Weise, daß ein Gemisch aus den Grundkomponenten, umfassend Wasserphase, Ölphase, einen oder mehrere der erfindungsgemäß verwendeten O/W-Emulgatoren, gewünschtenfalls einen oder mehrere W/O-Emulgatoren, sowie gewünschtenfalls weitere Hilfs-, Zusatz- und/oder Wirkstoffe, welche unterhalb des Phaseninversionstemperaturbereiches eine O/W-Emulsion bilden, auf eine Temperatur bringt,

- bei welcher die in der Ölphase löslichen Komponenten entweder gelöst oder zumindest in geschmolzenem Zustande vorliegen
- und welche mindestens der Schmelztemperatur der höchstschmelzenden, nicht in gelöstem Zustande vorliegenden Ölkomponente entspricht,
- welche unterhalb des Phaseninversionstemperaturbereiches des Systemes liegt,

und die entstandene O/W-Emulsion unter Bildung einer O/W Mikroemulsion hernach auf Raumtemperatur abkühlt. Dies geschieht bevorzugt unter Rühren.

Dieses erfindungsgemäße Verfahren ist besonders gut geeignet, wenn den erfindungsgemäßen O/W-Mikroemulsionen wärmeempfindliche oder leichtflüchtige Substanzen einverleibt werden sollen. Darüberhinaus ist dieses bei relativ niedrigen Temperaturen durchzuführende Verfahren gegenüber üblichen Verfahren energiesparend.

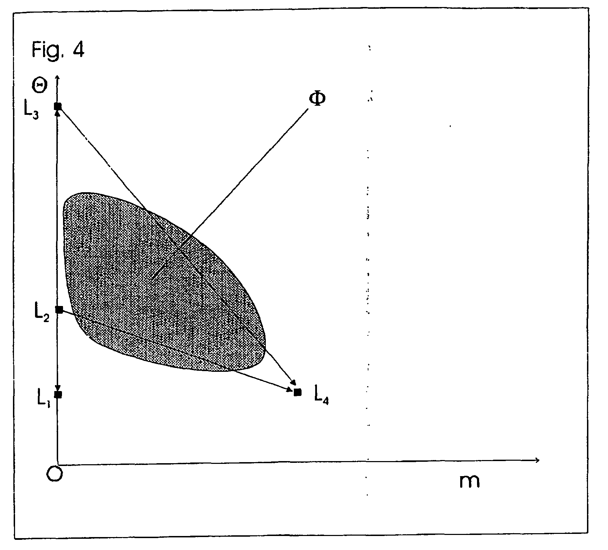

In Fig. 4 wird der Fall beschrieben, in welchem in der Koordinate $L_1$ zunächst kein erfindungsgemäßer

O/W-Emulgator vorliegt, und in welchem das System durch Erhöhen der Temperatur auf eine Koordinaten $L_3 \notin \Phi$ oder auf eine Koordinate $L_2 \notin \Phi$ gebracht wird. Selbstverständlich kann die Koordinate $L_2$ auch durch Abkühlen eines in der Koordinate $L_3$ vorliegenden Systems erreicht werden. Die Koordinaten $L_2$ und $L_3$, in denen beispielsweise W/O-Emulsionen vorliegen können, unterscheiden sich prinzipiell lediglich dadurch, daß die $L_3$ zugeordnete Temperatur höher ist als jede Temperatur, die dem Phaseninversionstemperaturbereiche zugeordnet werden kann.

Die Gegenwart eines zusätzlichen W/O-Emulgators für Systeme, die in Fig. 4 symbolisiert werden, ist nicht unbedingt erforderlich, allerdings vorteilhaft. Zugabe eines erfindungsgemäßen O/W-Emulgators oder mehrerer solcher Emulgatoren in den Koordinaten $L_2$ oder $L_3$, bei Senkung der Temperatur, befördert das System zur Koordinate $L_4$, bei welcher dann eine erfindungsgemäße O/W-Mikroemulsion vorliegt.

Eine weitere vorteilhafte Ausführungsform des erfindungsgemäßen Verfahrens besteht demgemäß darin, nach der Auswahl geeigneter Rohstoffe, d.h., Wasser- und Ölphase und gegebenenfalls weitere Substanzen, die Einzelkomponenten unter Rühren auf eine Temperatur zu bringen, bei welcher Phaseninversion für das gegebene Gemisch möglich ist, und durch Zugabe des erfindungsgemäß verwendeten O/W-Emulgators oder der erfindungsgemäß verwendeten O/W-Emulgatoren zum Gemisch Phaseninversion herbeizuführen, hernach unter fortwährendem Rühren das Gemisch auf Raumtemperatur abkühlen zu lassen.

Es übersteigt nicht das Können des Fachmannes, durch einfache Versuche den geeigneten Temperaturbereich zu ermitteln, innerhalb dessen ein gegebenes Gemisch Phaseninversion durchlaufen kann. Überlicherweise ist dieser Temperaturbereich zwischen 70 und 95° C zu wählen, kann im Einzelfalle auch darüber oder darunter angesiedelt sein.

In der Praxis ist möglich und gegebenenfalls sogar vorteilhaft, bei der Herstellung einer erfindungsgemäßen Mikroemulsion den Temperaturbereich, der dem Phaseninversionsbereich zugeordnet werden kann, auch zu übersteigen, da beim Abkühlen auf Raumtemperatur dieser Bereich dann zwangsläufig durchlaufen wird.

Der Zusatz von Elektrolyten bewirkt eine Veränderung des Löslichkeitsverhaltens eines hydrophilen Emulgators. Die erfindungsgemäßen Mikroemulsionen enthalten daher vorteilhaft Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraëssigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des oder der Elektrolyte sollte etwa 0,1 - 10,0 Gew.-%, besonders vorteilhaft etwa 0,3 - 8,0 Gew.% betragen, bezogen auf das Gesamtgewicht der Zubereitung.

Die erfindungsgemäßen Zubereitungen können in Form von Aërosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren Mikroemulsionen.

Als Treibmittel für erfindungsgemäße, aus Aërosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Es hat sich darüberhinaus in überraschender Weise herausgestellt, daß bei der Verwendung von in der Ölphase löslichen Treibmitteln, also beispielsweise üblichen Propan-Butan-Gemischen, die erfindungsgemäßen O/W-Mikroemulsionen nicht einfach als Aërosoltröpfchen versprüht werden, sondern sich zu feinblasigen, reichhaltigen Schäumen entwickeln, sobald solche mit solchen Treibmitteln beladenen Systeme Druckentspannung erfahren.

Solche nachschäumenden Zubereitungen werden daher ebenfalls als vorteilhafte Verkörperungen der vorliegenden Erfindung angesehen.

Bei der Verwendung von in der Ölphase unlöslichen Treibmitteln werden die erfindungsgemäßen O/W-Mikroemulsionen als Aërosoltröpfchen versprüht.

Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden. Es ist dabei vorteilhaft, Antioxidantien als einzige

Wirkstoffklasse zu verwenden, etwa dann, wenn eine kosmetische oder dermatologische Anwendung im Vordergrunde steht wie die Bekämpfung der oxidativen Beanspruchung der Haut. Es ist aber auch günstig, die erfindungsgemäßen Mikroemulsionen mit einem Gehalt an einem oder mehreren Antioxidantien zu versehen, wenn die Zubereitungen einem anderen Zwecke dienen sollen, z.B. als Desodorantien oder Sonnenschutzmittel.

Besonders vorteilhaft werden die Antioxidantien gewählt aus der Gruppe bestehend aus

Aminosäuren (z.B. Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin. Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze. Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptahioninsulfoximin) in sehr geringen verträglichen Dosierungen (z.B. pmol bis μmol/kg), ferner (Metall)-Chelatoren (z.B. α-Hydroxyfettsäuren, α-Hydroxypalmitinsäure, Phytinsäure, Lactoferrin), α-Hydroxysäuren (z.B. Zitronensäure, Milchsäure, Apfelsäure), Huminsäure, Gallensäure, Chlorogensäure, Kaffeesäure, Gallenextrakte, Bilirubin, Biliverdin, EDTA, EGTA und deren Derivate, ungesättigte Fettsäuren und deren Derivate (z.B. γ-Linolensäure. Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.%, insbesondere 1 - 10 Gew.-%, bezogen auf das Gesamtgewicht der Zubereitung.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Wenn es gewünscht wird, kann die Wasserphase der erfindungsgemäßen O/W-Mikroemulsionen auch Verdicker enthalten, so daß die Gesamtzubereitung gelartig erscheint und als Mikroemulsionsgel aufzufassen ist. Als geeignete Verdicker haben sich beispielsweise Carragheenan bzw. PEG-4-Rapeseedamide sowie Laureth-2 amid MEA herausgestellt.

Erfindungsgemäß können Wirkstoffe auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivate, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Obgleich selbstverständlich auch die Verwendung hydrophiler Wirkstoffe erfindungsgemäß begünstigt ist, ist ein weiterer Vorteil der erfindungsgemäßen Mikroemulsionen, daß die hohe Anzahl feinstzerteilter Tröpfchen gerade öllösliche bzw. lipophile Wirkstoffe mit besonders großer Wirksamkeit biologisch verfügbar macht.

Vorteilhaft ist es auch, die Wirkstoffe aus der Gruppe der rückfettenden Substanzen zu wählen, beispielsweise Purcellinöl, Eucerit® und Neocerit®.

Die erfindungsgemäßen Zubereitungen können kosmetische Hilfsstoffe enthalten, wie sie üblicherweise in solchen Zubereitungen verwendet werden, z.B. Konservierungsmittel, Bakterizide, Viruzide, Parfüme, Substanzen zum Verhindern des Schäumens. Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, Medikamente, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel.

Insbesondere vorteilhaft werden Gemische der vorstehend genannten Lösungsmittel verwendet.

Als weitere Bestandteile können verwendet werden Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkohofen mit Alkansäuren niedriger C-Zahl oder mit Fettsäuren, Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, - monoethyl- oder -monobutylether, Diethylenglykolmonomethyl- oder -monoethylether und analoge Produkte.

Die nachfolgenden Beispiele sollen die vorliegende Erfindung verdeutlichen.

**Beispiel 1:**

| | Gew.-% |
|---|---|
| Isoceteth-20 | 0,80 |
| Glycerylisostearat | 0,40 |
| Phenyl Trimethicon | 0,50 |
| Cetrimoniumchlorid | 0,35 |
| Parfüm u. Konservierungsmittel | q.s. |
| Wasser | ad 100,00 |

**Beispiel 2:**

| | Gew.-% |
|---|---|
| Glycerylisostearat | 0,33 |
| Isoceteth-15 | 0,93 |
| Ocytylisostearat | 0,60 |
| Cyclomethicon | 1,20 |
| Cetrimoniumchlorid | 0,40 |
| Parfüm u. Konservierungsmittel | q.s. |
| Wasser | ad 100,00 |

**Beispiel 3:**

| | Gew.-% |
|---|---|
| Sorbitanmonoisostearat | 0,42 |
| Isoceteth-15 | 0,84 |
| Isotridecylisononanoat | 0,60 |
| Cyclomethicon | 1,20 |
| Cetrimoniumchlorid | 0,40 |
| Parfüm u. Konservierungsmittel | q.s. |
| Wasser | ad 100,00 |

**Beispiel 4:**

| | |
|---|---|
| Diglycerylmonoisostearat | 0,42 |
| Isoceteth-15 | 0,84 |
| Isotridecylisononanoat | 0,60 |

Tabelle fortgesetzt

| | |
|---|---|
| Cyclomethicon | 1,20 |
| Cetrimoniumchlorid | 0,40 |
| Parfüm u. Konservierungsmittel | q.s. |
| Wasser | ad 100,00 |

**Beispiel 5:**

| | Gew.% |
|---|---|
| Glycerylisostearat | 0,44 |
| PEG-60-Evening Primrose Glyceride | 0,87 |
| Isotridecylisononanoat | 0,60 |
| Cyclomethicon | 1,20 |
| Cetrimoniumchlorid | 0,45 |
| Propylenglycol | 0,55 |
| Parfüm u. Konservierungsmittel | q.s. |
| Wasser | ad 100,00 |

**Beispiel 6:**

| | Gew.-% |
|---|---|
| Glycerylisolaurat | 0,83 |
| Laureth-11-carboxylsäure (90%) | 0,68 |
| Cetearylisononanoat | 0,32 |
| Cyclomethicon | 0,63 |
| Cetrimoniumchlorid | 0,40 |
| Parfüm u. Konservierungsmittel | q.s. |
| Wasser | ad 100,00 |

**Beispiel 7:**

| | Gew.-% |
|---|---|
| Glycerylisostearat | 0,44 |
| PEG-20-stearat | 0,87 |
| Isotridecylisononanoat | 0,60 |
| Cyclomethicon | 0.61 |
| Cetrimoniumchlorid | 0.38 |
| Parfüm u. Konservierungsmittel | q.s. |
| Wasser | ad 100,00 |

**Beispiel 8:**

| | Gew.-% |
|---|---|
| Glycerylisostearat | 0,44 |

Tabelle fortgesetzt

| | Gew.-% |
|---|---|
| Ceteareth-15 | 0,87 |
| Caprylic/Capric/Triglyceride | 0,60 |
| Cyclomethicon | 0,80 |
| Cetrimoniumchtorid | 0,40 |
| Parfüm u. Konservierungsmittel | q.s. |
| Wasser | ad 100,00 |

**Beispiel 9:**

| | Gew.-% |
|---|---|
| Glycerylisostearat | 0,34 |
| Ceteareth-15 | 0,77 |
| Dicaprylether | 0,80 |
| Cetrimoniumchlorid | 0,40 |
| Parfüm u. Konservierungsmittel | q.s. |
| Wasser | ad 100,00 |

**Beispiel 10:**

| | Gew.-% |
|---|---|
| Diglycerinmonoisostearat | 0,28 |
| Ceteareth-15 | 0,76 |
| Paraffinum liquidum | 0,70 |
| Cetrimoniumchlorid | 0,35 |
| Parfüm u. Konservierungsmittel | q.s. |
| Wasser | ad 100,00 |

**Beispiel 11:**

| | Gew.-% |
|---|---|
| Diglycerinmonoisostearat | 0,28 |
| Ceteareth-15 | 0,82 |
| Octyldodecanol | 0,80 |
| Cetrimoniumchlorid | 0,35 |
| Parfüm u. Konservierungsmittel | q.s. |
| Wasser | ad 100,00 |

**Beispiel 12:**

| | Gew.-% |
|---|---|
| PEG-6 Caprylsäure/Caprinsäureglyceride | 0,87 |
| Isotridecylisononanoat | 0,30 |

Tabelle fortgesetzt

| | Gew.-% |
|---|---|
| Cyclomethicon | 0,61 |
| Cetrimoniumchlorid | 0,35 |
| Parfüm u. Konservierungsmittel | q.s. |
| Wasser | ad 100,00 |

**Beispiel 13:**

| | Gew.% |
|---|---|
| Glycerylisostearat | 0,44 |
| Isotridecylisononanoat | 0,30 |
| PEG-20-Glycerylisostearat | 0,87 |
| Cyclomethicon | 0,60 |
| Cetrimoniumchloride | 0,40 |
| Parfüm u. Konservierungsmittel | q.s. |
| Wasser | ad 100,00 |

## Patentansprüche

**1.** Haarpflegezubereitungen, in Form einer transparenten oder transluzenten Mikroemulsion vom Typ Öl-in-Wasser, **die einen Emulgatorgehalt kleiner als 2,0 Gew.-%, bezogen auf das Gesamtgewicht der Emulsion, aufweisen,**

- umfassend eine Ölphase, und eine Wasserphase
- enthaltend:

einen oder mehreren polyethoxylierte O/W-Emulgatoren und/oder
einen oder mehrere polypropoxylierte O/W-Emulgatoren und/oder
einen oder mehrere polyethoxylierte und polypropoxylierte O/W-Emulgatoren, gewählt aus der Gruppe

- der Fettalkoholethoxylate der allgemeinen Formel R-O-(-$CH_2$-$CH_2$-O-)-H, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel R-O-(-$CH_2$-$CH_2$-O-)-R', wobei R und R' unabhängig voneinander verzweigte oder unverzweigte Alkyl- oder Alkenylreste und n eine Zahl von 10 bis 80 darstellen
- der Fettsäureethoxylate der allgemeinen Formel R-COO-(-$CH_2$-$CH_2$-O-)-H, wobei R einen verzweigten oder unverzweigten Alkyl- oder Alkenylrest und n eine Zahl von 10 bis 40 darstellen,
- der veretherten Fettsäureethoxylate der allgemeinen Formel R-COO-(-$CH_2$-$CH_2$-O-)-R', wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel R-COO-(-$CH_2$-$CH_2$-O-)-C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweiger Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel R-O-(-$CH_2$-$CH_2$-O-)-$CH_2$-COOH bzw. deren kosmetisch oder pharmazeutisch akzeptablen Salze, wobei R einen verzweigten oder unverzweigten Alkyloder Alkenylrest mit 5 - 30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel $R-O-(-CH_2-CH_2-O-)-SO_3-H$ mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen.
- der Fettalkohol propoxylate der allgemeinen Formel $R-O-(-CH_2-CH(CH_3)-O-)_n-H$, wobei R einen verzweigten oder unverzweigten Alkyl- oder Alkenylrest und n eine Zahl von 10 bis 80 darstellen,
- der Polypropylenglycolether der allgemeinen Formel $R-O-(-CH_2-CH(CH_3)-O-)_n-R'$, wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel $R-COO-(-CH_2-CH(CH_3)-O-)_n-R'$, wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel $R-COO-(-CH_2-CH(CH_3)-O-)_n-C(O)-R'$, wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel $R-COO-(-CH_2-CH(CH_3)-O-)_n-H$, wobei R einen verzweigten oder unverzweigten Alkyloder Alkenylrest und n eine Zahl von 10 bis 80 darstellen,
- der Polypropylenglycolglycerinfettsäureester gesättigter und/oder ungesättigter, verzweigter und/oder unverzweiger Fettsäuren und einem Propoxylierungsgrad zwischen 3 und 80
- der propoxylierten Sorbitanester mit einem Propoxylierungsgrad von 3 bis 100
- der Cholesterinpropoxylate mit einem Propoxylierungsgrad von 3 bis 100
- der propoxylierten Triglyceride mit einem Propoxylierungsgrad von 3 bis 100 der Alkylethercarbonsäuren der allgemeinen Formel $R-O-(-CH_2-CH(CH_3)O-)_n-CH_2-COOH$ bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel $R-O-(-CH_2-CH(CH_3)-O-)_n-SO_3-H$ mit kosmetisch oder pharmazeutisch akzeptablen Kationen, wobei R einen verzweigten oder unverzweigten Alkyl- oder Alkenylrest mit 5-30 C-Atomen und n eine Zahl von 1 bis 50 darstellen,
- der Fettalkoholethoxylate/propoxylate der allgemeinen Formel $R-O-X_n-Y_m-H$,wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel $R-O-X_n-Y_m-R'$, wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel $R-COO-X_n-Y_m-R'$, wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel $R-COO-X_n-Y_m-H$, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

**wobei der Anteil der polyethoxylierten bzw. der polypropoxylierten bzw. der polyethoxylierten und polypropoxylierten Emulgatoren unter 1,5 Gew.-%, bezogen auf das Gesamtgewicht der Zubereitung liegt**
- gewünschtenfalls ferner enthaltend einen oder mehrere W/O-Emulgatoren
- erhältlich durch ein Verfahren, dergestalt, daß

  - ein Gemisch aus den Grundkomponenten,
  - welches unterhalb des Phaseninversionstemperaturbereiches eine O/W-Emulsion bildet,

- auf eine Temperatur oberhalb oder innerhalb des Phaseninversionstemperaturbereiches gebracht,
- und die gebildete Mikroemulsion hernach auf Raumtemperatur abkühlt wird,

- ferner enthaltend mindestens ein kationischens Tensid, gewählt aus der Gruppe der quaternären Ammoniumverbindungen, sofern diese keine flimbildenden Eigenschaften besitzen, insbesondere Benzyldialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylam inoxide,

**wobei weniger als 0,5 Gew.-% an einem oder mehreren kationischen Tensiden vorliegen.**

2. Verwendung von Zubereitungen nach Anspruch 1 zur Erhöhung der Haarfülle, zur Verbesserung des Haarbodys und des Haarvolumens sowie des Haltes der Haartracht.

3. Zubereitungen nach Anspruch 1 oder Verwendung nach Anspruch 2, **dadurch gekennzeichnet, daß** die Ölphase gewählt wird aus der Gruppe der Silkonöle, insbesondere aus der Gruppe Phenyltrimethicon, Cyclomethicon (Octamethylcyclotetrasiloxan) Hexamethylcyclotrisiloxan, Polydimethylsiloxan, Poly(methylphenylsiloxan), ferner aus Cyclomethicon und Isotridecylisononanoat, sowie Mischungen aus Cyclomethicon und 2-Ethylhexylisostearat.

## Claims

1. Haircare preparations in the form of a transparent or translucent microemulsion of the oil-in-water type which have an emulsifier content of less than 2.0% by weight, based on the total weight of the emulsion,

- comprising an oil phase, and a water phase
- comprising:

one or more polyethoxylated O/W emulsifiers and/or one or more polypropoxylated O/W emulsifiers and/or one or more polyethoxylated and polypropoxylated O/W emulsifiers, chosen from the group consisting of

- fatty alcohol ethoxylates of the general formula $R-O-(-CH_2-CH_2-O-)-H$, where R is a branched or unbranched alkyl, aryl or alkenyl radical and n is a number from 10 to 50
- ethoxylated wool wax alcohols,
- polyethylene glycol ethers of the general formula $R-O-(-CH_2-CH_2-O-)-R'$, where R and R', independently of one another, are branched or unbranched alkyl or alkenyl radicals and n is a number from 10 to 80
- fatty acid ethoxylates of the general formula $R-COO-(-CH_2-CH_2-O-)-H$, where R is a branched or unbranched alkyl or alkenyl radical and n is a number from 10 to 40,
- etherified fatty acid ethoxylates of the general formula $R-COO-(-CH_2-CH_2-O-)-R'$, where R and R', independently of one another, are branched or unbranched alkyl or alkenyl radicals and n is a number from 10 to 80,
- esterified fatty acid ethoxylates of the general formula $R-COO-(-CH_2-CH_2-O-)-C(O)-R'$, where R and R', independently of one another, are branched or unbranched alkyl or alkenyl radicals and n is a number from 10 to 80,
- polyethylene glycol glycerol fatty acid esters of saturated and/or unsaturated, branched and/or unbranched fatty acids and a degree of ethoxylation between 3 and 50,
- ethoxylated sorbitan esters with a degree of ethoxylation of from 3 to 100
- cholesterol ethoxylates with a degree of ethoxylation between 3 and 50,
- ethoxylated triglycerides with a degree of ethoxylation between 3 and 150,
- alkyl ether carboxylic acids of the general formula $R-O-(-CH_2-CH_2-O-)-CH_2-COOH$ and cosmetically or pharmaceutically acceptable salts thereof, where R is a branched or unbranched alkyl or alkenyl radical having 5-30 carbon atoms and n is a number from 5 to 30,
- polyoxyethylene sorbitol fatty acid esters based on branched or unbranched alkanoic or alkenoic acids and having a degree of ethoxylation of from 5 to 100, for example of the sorbeth type,
- alkyl ether sulphates and the acids on which these sulphates are based of the general formula

$R-O-(-CH_2-CH_2-O-)-SO_3-H$ with cosmetically or pharmaceutically acceptable cations, where R is a branched or unbranched alkyl or alkenyl radical having 5-30 carbon atoms and n is a number from 1 to 50,
- fatty alcohol propoxylates of the general formula $R-O-(-CH_2-CH(CH_3)-O-)_n-H$, where R is a branched or unbranched alkyl or alkenyl radical and n is a number from 10 to 80,
- polypropylene glycol ethers of the general formula $R-O-(-CH_2-CH(CH_3)-O-)_n-R'$, where R and R', independently of one another, are branched or unbranched alkyl or alkenyl radicals and n is a number from 10 to 80
- propoxylated wool wax alcohols,
- etherified fatty acid propoxylates of the general formula $R-COO-(-CH_2-CH(CH_3)-O-)_n-R'$, where R and R', independently of one another, are branched or unbranched alkyl or alkenyl radicals and n is a number from 10 to 80,
- esterified fatty acid propoxylates of the general formula $R-COO-(-CH_2-CH(CH_3)-O-)_n-C(O)-R'$, where R and R', independently of one another, are branched or unbranched alkyl or alkenyl radicals and n is a number from 10 to 80,
- fatty acid propoxylates of the general formula $R-COO-(-CH_2-CH(CH_3)-O-)_n-H$, where R is a branched or unbranched alkyl or alkenyl radical and n is a number from 10 to 80,
- polypropylene glycol glycerol fatty acid esters of saturated and/or unsaturated, branched and/or unbranched fatty acids and a degree of propoxylation between 3 and 80
- propoxylated sorbitan esters with a degree of propoxylation of from 3 to 100
- cholesterol propoxylates with a degree of propoxylation of from 3 to 100
- propoxylated triglycerides with a degree of propoxylation of from 3 to 100
- alkyl ether carboxylic acids of the general formula $R-O-(-CH_2-CH(CH_3)O-)_n-CH_2-COOH$ and cosmetically or pharmaceutically acceptable salts thereof, where R is a branched or unbranched alkyl or alkenyl radical and n is a number from 3 to 50,
- alkyl ether sulphates and the acids on which these sulphates are based of the general formula $R-O-(-CH_2-CH(CH_3)-O-)_n-SO_3-H$ with cosmetically or pharmaceutically acceptable cations, where R is a branched or unbranched alkyl or alkenyl radical having 5-30 carbon atoms and n is a number from 1 to 50,
- fatty alcohol ethoxylates/propoxylates of the general formula $R-O-X_n-Y_m-H$, where R is a branched or unbranched alkyl or alkenyl radical, where X and Y are not identical and are in each case either an oxyethylene group or an oxypropylene group and n and m, independently of one another, are numbers from 5 to 50,
- polypropylene glycol ethers of the general formula $R-O-X_n-Y_m-R'$, where R and R', independently of one another, are branched or unbranched alkyl or alkenyl radicals, where X and Y are not identical and are in each case either an oxyethylene group or an oxypropylene group and n and m, independently of one another, are numbers from 5 to 100,
- etherified fatty acid propoxylates of the general formula $R-COO-X_n-Y_m-R'$, where R and R', independently of one another, are branched or unbranched alkyl or alkenyl radicals, where X and Y are not identical and are in each case either an oxyethylene group or an oxypropylene group and n and m, independently of one another, are numbers from 5 to 100,
- fatty acid ethoxylates/propoxylates of the general formula $R-COO-X_n-Y_m-H$, where R is a branched or unbranched alkyl or alkenyl radical, where X and Y are not identical and are in each case either an oxyethylene group or an oxypropylene group and n and m, independently of one another, are numbers from 5 to 50,

where the fraction of the polyethoxylated or of the polypropoxylated or of the polyethoxylated and polypropoxylated emulsifiers is less than 1.5% by weight, based on the total weight of the preparation
- if desired also comprising one or more W/O emulsifiers
- obtainable by a process in which

  - a mixture of the base components,

    - which forms an o/w emulsion below the phase inversion temperature range,
    - is brought to a temperature above or within the phase inversion temperature range,
    - and the formed microemulsion is subsequently cooled to room temperature,

  - also comprising at least one cationic surfactant chosen from the group of quaternary ammonium compounds provided these have no film-forming properties, in particular benzyldialkylammonium chlorides or

bromides, such as, for example, benzyldimethylstearylammonium chloride, also alkyltrialkylammonium salts, for example cetyltrimethylammonium chloride or bromide, alkyldimethylhydroxyethylammonium chlorides or bromides, dialkyldimethylammonium chlorides or bromides, alkylamidoethyltrimethylammonium ether sulphates, alkylpyridinium salts, for example lauryl- or cetylpyrimidinium chloride, imidazoline derivatives and compounds with cationic character, such as amine oxides, for example alkyldimethylamine oxides or alkylaminoethyldimethylamine oxides,

where less than 0.5% by weight of one or more cationic surfactants is present.

**2.** Use of preparations according to Claim 1 for increasing hair fullness, for improving hair body and hair volume, and also hold of the hairstyle.

**3.** Preparations according to Claim 1 or use according to Claim 2, **characterized in that** the oil phase is chosen from the group of silicone oils, in particular from the group consisting of phenyltrimethicone, cyclomethicone (octamethylcyclotetrasiloxane) hexamethylcyclotrisiloxane, polydimethylsiloxane, poly(methylphenylsiloxane), also cyclomethicone and isotridecyl isononanoate, and mixtures of cyclomethicone and 2-ethylhexyl isostearate.

## Revendications

**1.** Préparations de soins des cheveux, sous la forme d'une micro émulsion transparente ou translucide du type huile-dans-eau, qui présentent une teneur en émulsifiant inférieure à 2,0 % en poids, par rapport au poids total de l'émulsion,

- comprenant une phase huileuse et une phase aqueuse
- contenant:

un ou plusieurs émulsifiants H/E polyéthoxylés et/ou
un ou plusieurs émulsifiants H/E polypropoxylés et/ou un ou plusieurs émulsifiants H/E polyéthoxylés et polypropoxylés, sélectionnés parmi le groupe

- des éthoxylates d'alcools gras de la formule générale R-O-(-$CH_2$-$CH_2$-O-)-H, R représentant un radical alkyle, aryle ou alcényle, ramifié ou non ramifié, et n représentant un nombre de 10 à 50,
- des alcools de graisse de laine éthoxylés, -des éthers de polyéthylèneglycol de la formule générale R-O-(-$CH_2$-$CH_2$-O-)-R', R et R' représentant, indépendamment l'un de l'autre, un radical alkyle, ou alcényle, ramifié ou non ramifié, et n représentant un nombre de 10 à 80,
- des éthoxylates d'acides gras de la formule générale R-COO-(-$CH_2$-$CH_2$-O-)-H, R représentant un radical alkyle ou alcényle, ramifié ou non ramifié, et n représentant un nombre de 10 à 40,
- des éthoxylates d'acides gras éthérés de la formule générale R-COO-(-$CH_2$-$CH_2$-O-)-R', R et R' représentant, indépendamment l'un de l'autre, un radical alkyle ou alcényle, ramifié ou non ramifié et n représentant un nombre de 10 à 80,
- des éthoxylates d'acides gras estérifiés de la formule générale R-COO-(-$CH_2$-$CH_2$-O-)-C(O)-R', R et R' représentant, indépendamment l'un de l'autre, un radical alkyle ou alcényle, ramifié ou non ramifié, et n représentant un nombre de 10 à 80,
- des esters d'acides gras de la glycérine du polyéthylèneglycol et/ou des acides gras saturés et/ou insaturés, ramifiés et/ou non ramifiés, et ayant un degré d'éthoxylation compris entre 3 et 50,
- des esters de sorbitane éthoxylés ayant un degré d'éthoxylation allant de 3 à 100,
- des éthoxylates de cholestérine ayant un degré d'éthoxylation compris entre 3 et 50,
- des triglycérides éthoxylés ayant un degré d'éthoxylation compris entre 3 et 150,
- des acides carboxyliques d'éthers alkyles de la formule générale R-O-(-$CH_2$-$CH_2$-O-)$CH_2$-COOH ou leurs sels acceptables du point de vue cosmétique ou pharmaceutique, R représentant un radical alkyle ou alcényle, ramifié ou non ramifié, ayant 5 - 30 atomes de C et n représentant un nombre de 5 à 30,
- des esters d'acides gras de sorbitol de polyoxyéthylène, basés sur des acides d'alcanes ou d'alcènes et présentant un degré d'éthoxylation de 5 à 100, par exemple, du type Sorbeth,
- des sulfates d'éthers alkyles ou des acides formant la base de ces sulfates, de la formule générale R-O-(-$CH_2$-$CH_2$-O-)-$SO_3$-H avec des cations acceptables du point de vue cosmétique ou pharmaceutique, R représentant un radical alkyle ou alcényle, ramifié ou non ramifié, ayant 5 - 30 atomes de C et n représentant un nombre de 1 à 50,

- des propoxylates d'alcools gras de la formule générale $R-O-(-CH_2-CH(CH_3)-O-)_n-H$, R représentant un radical alkyle ou alcényle, ramifié ou non ramifié, et n représentant un nombre de 10 à 80,
- des éthers de polyéthylèneglycol de la formule générale $R-O-(-CH_2-CH(CH_3)-O-)_n-R'$, R et R' représentant, indépendamment l'un de l'autre, un radical alkyle ou alcényle, ramifié ou non ramifié, et n représentant un nombre de 10 à 80,
- des alcools de graisse de laine propoxylés,
- des propoxylates d'acides gras éthérifiés de la formule générale $R-COO-(-CH_2-CH(CH_3)-O-)_n-R'$, R et R' représentant, indépendamment l'un de l'autre, un radical alkyle ou alcényle, ramifié ou non ramifié, et n représentant un nombre de 10 à 80
- des propoxylates d'acides gras estérifiés de la formule générale $R-COO-(-CH_2-CH(CH_3)-O-)_n-C(O)-R'$, R et R' représentant, indépendamment l'un de l'autre, un radical alkyle ou alcényle, ramifié ou non ramifié, et n représentant un nombre de 10 à 80,
- des propoxylates d'acides gras de la formule générale $R-COO-(-CH_2-CH(CH_3)-O-)_n-H$, R représentant un radical alkyle ou alcényle, ramifié ou non ramifié, et n représentant un nombre de 10 à 80,
- des esters d'acides gras de la glycérine du polyéthylèneglycol et/ou des acides saturés et/ou insaturés, ramifiés et/ou non ramifiés et ayant un degré de propoxylation compris entre 3 et 80,
- des esters de sorbitane propoxylés, ayant un degré de propoxylation allant de 3 à 100,
- des propoxylates de la cholestérine ayant un degré de propoxylation de 3 à 100,
- des triglycérides propoxylées, ayant un degré de propoxylation de 3 à 100, des acides carboxyliques d'éthers alkyles de la formule générale $R-O-(-CH_2-CH(CH_3)O-)_n-CH_2-COOH$ ou leurs sels acceptables du point de vue cosmétique ou pharmaceutique, R représentant un radical alkyle ou alcényle, ramifié ou non ramifié et n représentant un nombre de 3 à 50,
- des sulfates d'éthers alkyles ou des acides formant la base de ces sulfates de la formule générale $R-O-(-CH_2-CH(CH_3)-O-)_n-SO_3-H$ avec des cations acceptables du point de vue cosmétique ou pharmaceutique, R représentant un radical alkyle ou alcényle, ramifié ou non ramifié, ayant 5 - 30 atomes de C et n représentant un nombre de 1 à 50,
- des éthoxylates/des propoxylates d'alcools gras de la formule générale $R-O-X_n-Y_m-H$, R représentant un radical alkyle ou alcényle, ramifié ou non ramifié, X et Y n'étant pas identiques et représentant, à chaque fois ou un groupement oxyéthylène ou un groupement oxypropylène, et n et m, représentant, indépendamment l'un de l'autre, des nombres de 5 à 50,
- des éthers du polypropylèneglycol de la formule générale $R-O-X_n-Y_m-R'$, R et R' représentant, indépendamment l'un de l'autre, un radical alkyle ou alcényle, ramifié ou non ramifié, X et Y n'étant pas identiques et représentant, à chaque fois ou un groupement oxyéthylène ou un groupement oxypropylène, et n et m, représentant, indépendamment l'un de l'autre, des nombres de 5 à 100,
- des propoxylates d'acides gras éthérés de la formule générale $R-COO-X_n-Y_m-R'$, R et R' représentant, indépendamment l'un de l'autre, un radical alkyle ou alcényle, ramifié ou non ramifié, X et Y n'étant pas identiques et représentant, à chaque fois ou un groupement oxyéthylène ou un groupement oxypropylène, et n et m, représentant, indépendamment l'un de l'autre, des nombres de 5 à 100,
- des éthoxylates/des propoxylates d'acides gras de la formule générale $R-COO-X_n-Y_m-H$, R représentant un radical alkyle ou alcényle, ramifié ou non ramifié, X et Y n'étant pas identiques et représentant, à chaque fois ou un groupement oxyéthylène ou un groupement oxypropylène, et n et m, représentant, indépendamment l'un de l'autre, des nombres de 5 à 50,

la proportion des émulsifiants polyéthoxylés ou polypropoxylés ou polyéthoxylés et polypropoxylés se situant en dessous de 1,5 % en poids, par rapport au poids total de la préparation,
- contenant, si on le souhaite, en outre, un ou plusieurs émulsifiants E/H
- que l'on peut obtenir par un procédé réalisé de telle sorte que

un mélange des composants de base,
- qui forme, en-dessous du domaine de température d'inversion de phases, une émulsion H/E,
- est amené à une température au-dessus ou au sein du domaine de température d'inversion de phases,
- et que la micro émulsion formée est refroidie ci-après à la température ambiante,

contenant en outre au moins un agent tensioactif cationique, sélectionné parmi le groupe des composés d'ammonium quaternaires, dans la mesure où ceux-ci ne possèdent aucune propriété filmogène, en particulier les chlorures ou les bromures de benzyldialkylammonium, comme, par exemple, le chlorure de benzyldiméthylstéarylammonium, en outre, les sels d'alkyltrialkylammonium, par exemple, le chlorure ou le bromure de cétyltriméthylammonium, les chlorures ou les bromures d'alkyldiméthylhydroxyéthylammonium, les chlorures ou les bromures de dialkyldiméthylammonium, les éthersulfates d'alkylamidéthyltriméthylammonium, les sels d'alkylpyridinium, par exemple, le

chlorure de lauryl- ou de cétyl-pyrimidinium, des dérivés de l'imidazole et des composés ayant un caractère cationique, comme les amino-oxydes, par exemple, les amino-oxydes d'alkyldiméthyle ou les amino-oxydes d'alkylaminoéthyldiméthyle, moins de 0,5 % en poids d'un ou de plusieurs des agents tensioactifs cationiques étant présent.

**2.** Utilisation de préparations selon la revendication 1, en vue de l'augmentation de la plénitude des cheveux, en vue de l'amélioration du corps des cheveux et du volume des cheveux ainsi que du maintien de la chevelure.

**3.** Préparations selon la revendication 1 ou utilisation selon la revendication 2, **caractérisées en ce que** la phase huileuse est sélectionnée parmi le groupe des huiles de silicone, en particulier parmi le groupe du phényltriméthicone, du cyclométhicone, (octaméthylcyclotétrasiloxane), de l'hexaméthylcyclotrisiloxane, du polydiméthylsiloxane, du poly(méthylphénylsiloxane), en outre du cyclométhicone et de l'isononanoate d'isotridécyle, ainsi que parmi des mélanges de cyclométhicone et d'isostéarate de 2-éthylhexyle.

Fig. 1

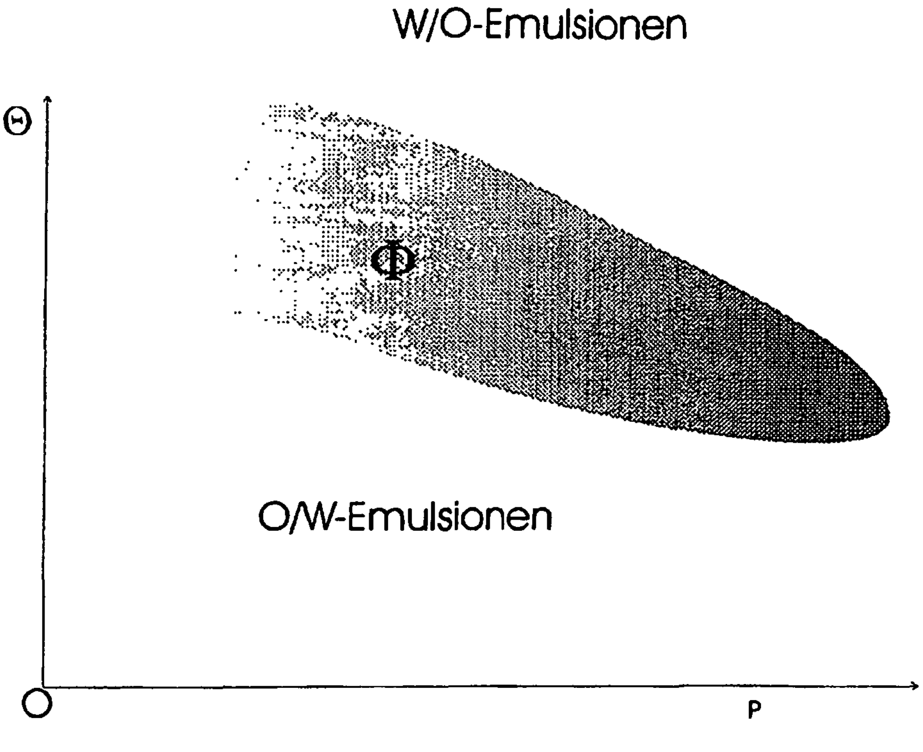

Fig. 2

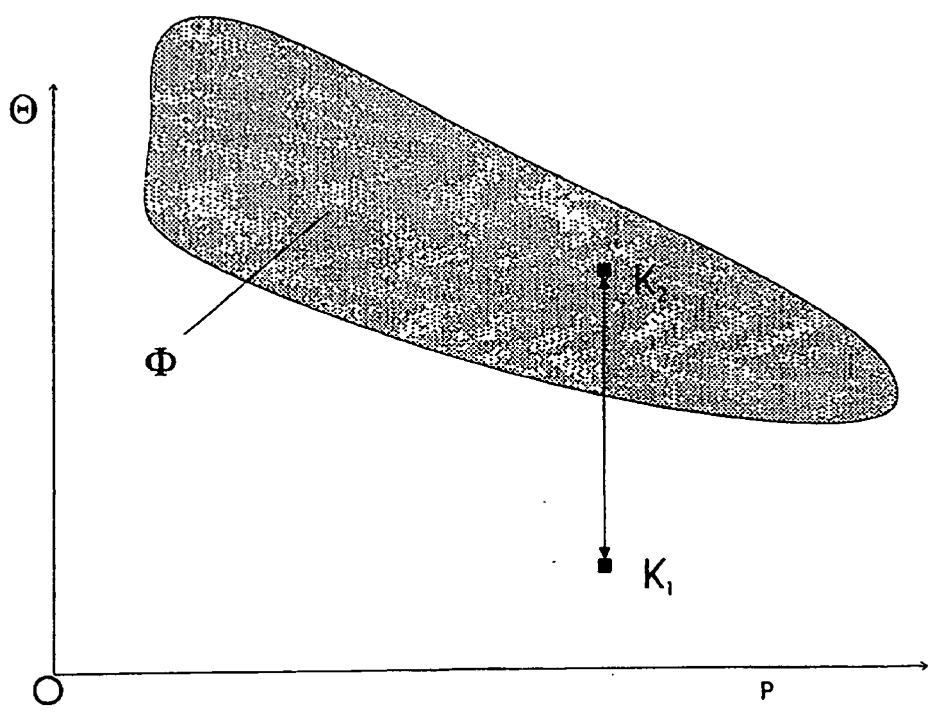

Fig. 3

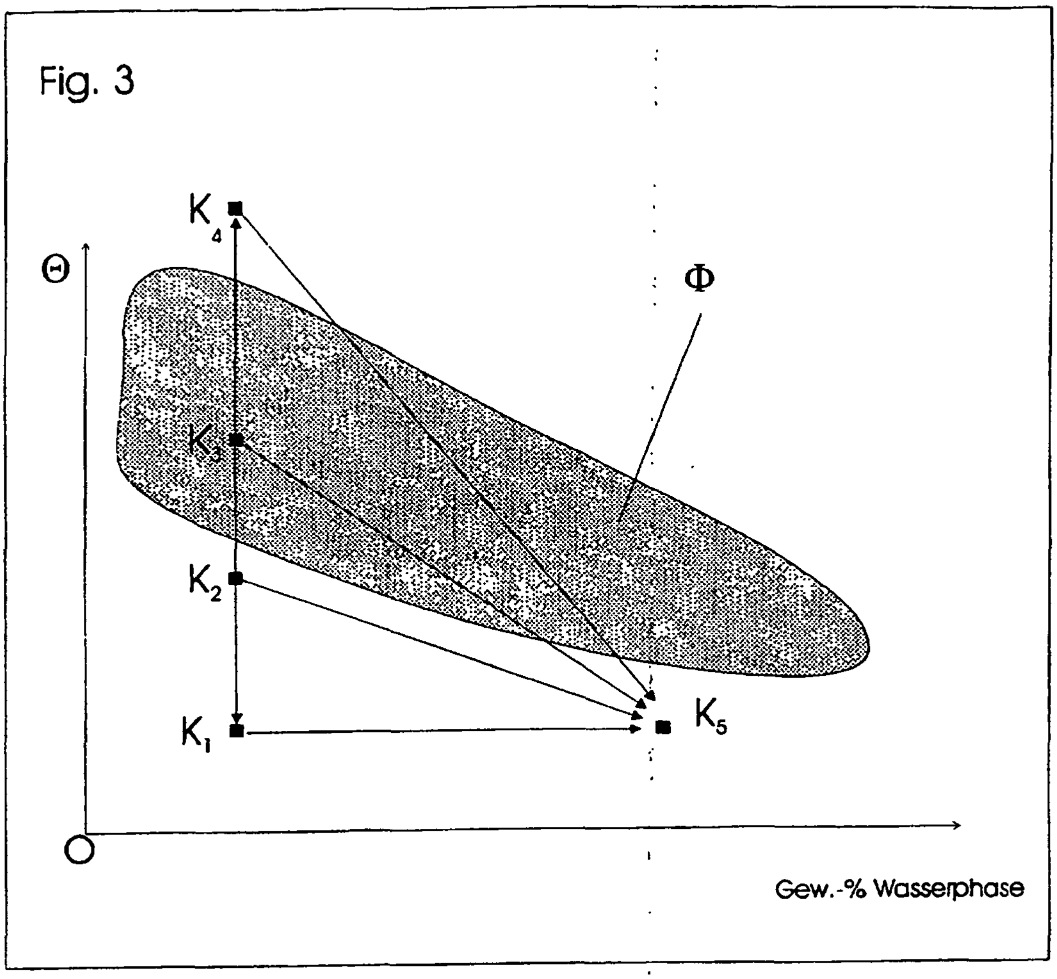

Fig. 4